(19) Europäisches Patentamt
European Patent Office
Office européen des brevets

(11) Publication number: **0 006 606**
**B1**

(12) **EUROPEAN PATENT SPECIFICATION**

(45) Date of publication of patent specification: **29.12.82**

(51) Int. Cl.$^3$: **C 07 D 209/88,**
C 07 D 209/86

(21) Application number: **79102111.6**

(22) Date of filing: **26.06.79**

(54) **Process for the preparation of carbazoles and intermediates therefor.**

(30) Priority: **26.06.78 US 919010**

(43) Date of publication of application:
**09.01.80 Bulletin 80/1**

(45) Publication of the grant of the patent:
**29.12.82 Bulletin 82/52**

(84) Designated Contracting States:
**AT CH LU NL SE**

(56) References cited:
**DE - A - 2 337 340**
**US - A - 3 896 145**

(73) Proprietor: **F. HOFFMANN-LA ROCHE & CO. Aktiengesellschaft**
**CH-4002 Basel (CH)**

(72) Inventor: **Berger, Leo**
**7 The Parkway**
**Montclair New Jersey (US)**
Inventor: **Corraz, Alfred John**
**17 Valley View Terrace**
**Wayne New Jersey (US)**
Inventor: **Parrish, David Richard**
**40 Stephen Street**
**Glen Ridge New Jersey (US)**
Inventor: **Scott, John William**
**81 Edgemont Road**
**Upper Montclair New Jersey (US)**

(74) Representative: **Lederer, Franz, Dr. et al,**
**Patentanwälte Dr. Franz Lederer Reiner F. Meyer**
**Lucile-Grahn-Strasse 22**
**D-8000 München 80 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of carbazoles and intermediates therefore

The invention relates to a process for the preparation of 6-chloro-$\alpha$-methylcarbazole-2-acetic acid, a compound useful as anti-inflammatory and analgesic agent as described in the DE—A—2 337 340 and the US—A—3 896 145. This process comprises:

a) either treating the compound of the formula

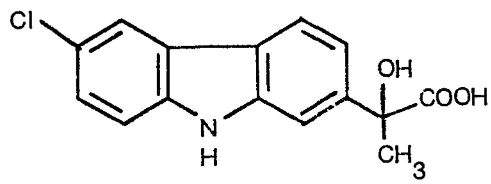

IIIb

with a dehydrating agent in an inert organic solvent and

b) hydrogenating the obtained compound of formula

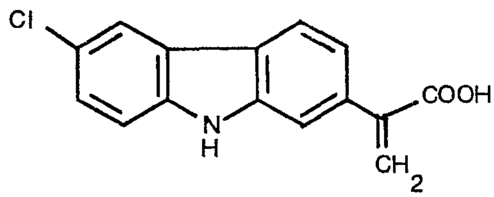

IV

or

c) subjecting the compound of formula IIIb to hydrogenolysis and

d) recovering the product of step b) or c) of the formula

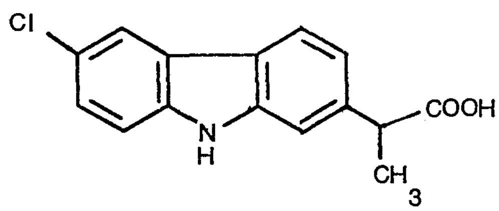

V

Steps a) and b) (IIIb→IV→V)

The 6-chloro-$\alpha$-hydroxy-$\alpha$-methylcarbazole-2-acetic acid of formula IIIb is treated with a dehydrating agent, e.g. an inorganic acid, such as hydrochloric or sulfuric acid, in an inert organic solvent, such as tetrahydrofuran. Conveniently, the dehydration is carried out at the reflux temperature of the reaction mixture. Subsequently, the resulting 6-chloro-$\alpha$-methylene-2-carbazole acetic acid is treated with a hydrogenating agent, such as hydrogen and platinum oxide in an alkanol, such as methanol or ethanol, to yield 6-chloro-$\alpha$-methylcarbazole-2-acetic acid of formula V.

Step c) (IIIb→V)

The 6-chloro-$\alpha$-methylcarbazole-2-acetic acid of formula IIIb can be subjected to hydrogenolysis utilizing, e.g. an organic acid, such as a lower alkanoic acid, e.g. glacial acetic acid or propionic acid, and an inorganic acid, such as hydrochloric acid, and a compound, such as stannous chloride, to yield 6-chloro-$\alpha$-methylcarbazole-2-acetic acid in a single step.

the compound of formula V, which is useful as an anti-inflammatory analgesic agent can be recovered from the reaction mixture by conventional separation methods, such as filtration.

The invention further relates to a process for the preparation of the above compound of the formula IIIb, which process comprises

a) chlorinating a compound of the formula

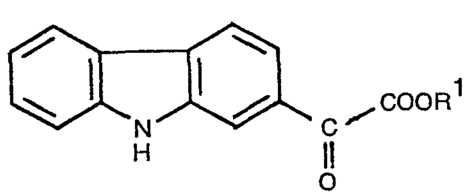

IIb

wherein $R^1$ is methyl or ethyl, in an inert organic solvent at a temperature, in the range of from about −40 to −60°C and either

b) treating the obtained compound of formula

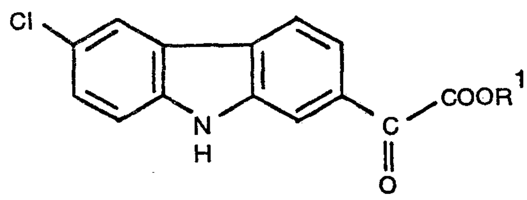

IIc

wherein $R^1$ is as above,

with a methyl Grignard reagent or with methyllithium in an inert organic solvent, and

c) treating the obtained compound of formula

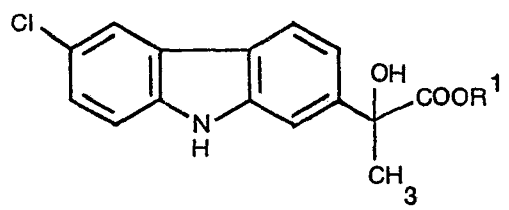

IIIa

wherein $R^1$ is as above,

with a base in an inert organic solvent, or

d) treating the ester of formula IIc with a base and

e) treating the obtained acid of formula

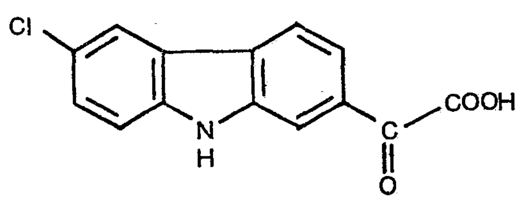

IId

with a methyl Grignard reagent or with methyllithium.

Step a) (IIb→IIc)

The carbazole-2-oxalic acid methyl or ethyl ester of formula IIb is chlorinated or treated with a chlorinating agent, such as sulfuryl chloride in an inert organic solvent, such as dimethyl formamide or 1,2-dichloroethane. The chlorination can be carried out at a temperature in the range of from about −40° to about −60°C. At low temperatures, selective chlorination at the 6-position occurs.

Steps b) and c) (IIc→IIIa→IIIb)

The 6-chlorcarbazole-2-oxalic acid methyl or ethyl ester of formula IIc, is reacted with a methyl Grignard reagent, e.g. a methylmagnesium halide, such as methylmagnesium iodide or bromide, or methyllithium, in the presence of an inert organic solvent, such as tetrahydrofuran or ether, preferably at a low temperature, e.g. in the range of 0 to 10°C.

The resulting 6-chloro-α-hydroxy-α-methylcarbazole-2-acetic acid methyl or ethyl ester of formula III is a hydrolyzed with base, e.g. with an alkali metal hydroxide, such as sodium or potassium hydroxide, in an inert organic solvent, e.g. an alkanol, such as ethanol or methanol, to yield the 6-chloro-α-hydroxy-α-methylcarbazole-2-acetic acid of formula IIIb. Conveniently, the hydrolysis is effected at the reflux temperature of the reaction mixture.

Steps d) and e) (IIc→IId→IIIb)

The 6-chlorocarbazole-2-oxalic acid methyl or ethyl ester of formula IIc can first be subjected to hydrolysis, as described above for the compound of formula IIIa, to obtain the 6-chlorocarbazole-2-oxalic acid of the formula IId, which is then treated with a methyl Grignard reagent or methyllithium, as discussed above for the conversion of the compound of formula IIc to that of formula IIIa. The obtained 6-chloro-α-hydroxy-α-methylcarbazole-2-acetic acid of formula IIIb can be separated by conventional procedures or can be utilized in situ in the reaction steps which follow.

The above compound of formula IIb can be prepared by a process which comprises
a) lower alkanoylating carbazole,
b) converting the obtained 9-lower alkanoylcarbazole of formula

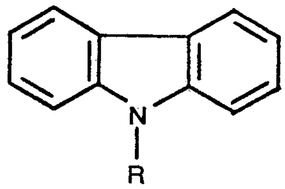

I

wherein R is lower alkanoyl,
into the corresponding 9-lkower alkanoylcarbazole-2-oxalic acid methyl or ethyl ester, and
c) de-lower alkanoylating the obtained compound of formula

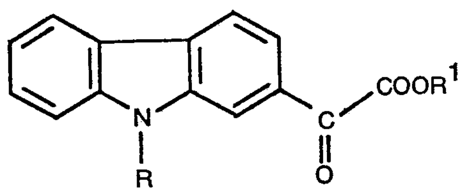

IIa

wherein R is as above and $R^1$ is methyl or ethyl.

Step a) (carbazole→I)

Carbazole is treated with a lower alkanoylating agent, e.g. an alkanoic anhydride, such as acetic or propionic anhydride, in an inert organic solvent, e.g. a halogenated alkane, such as methylene chloride, or chloroform in the presence of an inorganic acid, e.g. hydrochloric, hydrobromic or sulfuric acid. The reaction is conveniently carried out at the reflux temperature of the reaction mixture.

Step b) (I→IIa)

The 9-lower alkanoylcarbazole of formula I is converted to the corresponding oxalate of formula IIa utilizing, e.g. a methyl or ethyl oxalyl halide, such as ethyl oxalyl chloride, in an inert organic solvent, e.g. a halogenated alkane, such as methylene chloride, in the presence of a Lewis acid, e.g. aluminum chloride, titanium tetrachloride or boron trifluoride. The reaction temperature of this step is preferably in the range of from about 0 to 50°C, most preferably in the range of from about 0 to about 25°C.

Step c) (IIa→IIb)

The 9-lower alkanoylcarbazole-2-oxalic acid methyl or ethyl ester of formula IIa is deacylated utilizing, e.g. an inorganic acid, such as sulfuric or hydrochloric acid, in an alkanol, such as methanol or ethanol, to yield the corresponding carbazole-2-oxalic acid methyl or ethyl ester of formula IIb. The deacylation is preferably carried out at a temperature in the range of about 10 to about 80°C.

In each of the reaction steps of the above processes for preparing the compounds of formulae IIb, IIIb and V the intermediates which are produced may be separated or may be reacted in situ in the next reaction step., Also, the ratio of reactants is not critical. Preferably, however equimolar ratios are utilized.

The invention further relates to the novel intermediates of formulae IIb, IIc, IId, IIIa and IIIb and to the intermediate, 6-chloro-α-methylenecarbazole-2-acetic acid.

Example 1

To a stirred suspension of 167.2 g of carbazole in 400 ml of methylene chloride was added 103 ml of acetic anhydride and 2.0 ml of concentrated sulfuric acid. The mixture was degassed, placed under nitrogen and heated at reflux for 20 hours. The resulting solution was poured, after cooling to 20°, into a stirred solution of 175 g of sodium bicarbonate in 1600 ml of water. The mixture was stirred for 30 minutes and then the aqueous layer was extracted with 200 ml of methylene chloride. The combined organic solutions were washed with 200 ml of saturated sodium bicarbonate solution and dried over sodium sulfate. The sodium sulfate was removed by filtration and the filtrate was stirred for 1.0 hour with calcium chloride and 10 g of Carbon black. The mixture was filtered and washed with 400 ml of fresh methylene chloride to give a total filtrate of 1000 ml of a 1M solution of 9-acetylcarbazole. Such solutions appear to be stable indefinitely.

A stirred suspension of 600 g of aluminum chloride in 2000 ml of methylene chloride was degassed, placed under nitrogen and cooled to 3° in an ice bath. A mixture of the 9-acetylcarbazole

solution and 134 ml of ethyl oxalyl chloride was added within 2.0 hours so that an internal temperature of 5° was maintained. The nitrogen inlet was disconnected and the system was vented to a gas scrubber to remove the hydrogen chloride gas. The ice bath was replaced by a 20° water bath. The bath temperature was raised to 45° and stirring was maintained smooth over a 15 minute period. The internal temperature rose to 35° reflux, which was maintained for 1.0 hour.

2000 ml of ethanol was added within 1.0 hour, so that reflux was maintained. The mixture was then treated with 4000 ml of 6N hydrochloric acid within 1.0 hour, so that reflux continued. The two phase mixture was heated at reflux for 1.0 hour and cooled. The aqueous layer was extracted with 500 ml of methylene chloride and the combined organic solutions were filtered. Solvent removal gave crude crystalline 9-acetylcarbazole-2-glyoxalic acid ethyl ester.

A mixture of the crude 9-acetylcarbazole-2-glyoxalic acid ethyl ester, 1000 ml of ethanol and 100 ml of 6N sulfuric acid was degassed, placed under nitrogen and heated at reflux for 1.5 hour. The homogeneous mixture was cooled to 20° and treated with 3000 ml of water over a period of 1.0 hour. The resultant suspension was stirred an additional 30 minutes and filtered. The solid was washed with 3000 ml of water and dried. The resulting powder was heated at reflux with 3000 ml of chloroform. A small amount of insoluble material and a small aqueous layer were observed. The mixture was filtered and extracted with 2000 ml of 0.5N sodium bicarbonate solution. The chloroform solution was stirred for 10 minutes with sodium sulphate and 25 g of carbon black, filtered and evaporated at 60° to a volume of 800 ml. The mixture was warmed to effect solution and then 3200 ml of n-hexane was added under stirring over 1.0 hour at ambient temperature. The resultant suspension was stirred at ambient temperature for 16 hours and filtered. The solid was washed with 1000 ml of 4:1 n-hexane-chloroform. Drying gave 181 g of carbazole-2-glyoxalic acid ethyl ester, m.p. 130.5—131.5°.

Example 2

A solution of 267.28 g of carbazole-2-glyoxalic acid ethyl ester in 2000 ml of dimethylformamide was degassed, placed under nitrogen and cooled to —42°. 95 ml of sulfuryl chloride was added over 1.25 hours as the temperature was maintained at —42° to —45°. The temperature was allowed to rise to about 0° over 1.0 hour and the solution was treated, over 1.0 hour with 5000 ml of water. The resultant suspension was stirred for 30 minutes and filtered. The solid was washed with 2000 ml of water and dried for 16 hours. The crude 6-chlorocarbazole-2-glyoxalic acid ethyl ester was taken up in 9500 ml of chloroform at reflux, treated with 75 g of carbon black, filtered and washed with 2500 ml hot chloroform. The combined filtrates were distillated to a volume of 7600 ml. The solution was diluted with 7600 ml of n-hexane, stirred at ambient temperature for 16 hours and filtered. The solid was washed with 600 ml of 1:1 chloroform-n-hexane and dried to give 228 g of 6-chlorocarbazole-2-glyoxalic acid ether ester, m.p. 172.5—173°.

Example 3

A mixture of 75.43 g of 6-chlorocarbazole-2-glyoxalic acid ethyl ester and 25 ml of methanol was treated with a solution of 19.6 g of potassium hydroxide in 375 ml of water. The suspension was degassed, placed under nitrogen, stirred and heated at reflux for 1.0 hour to give an orange solution. Heating was stopped and the solution was treated with 200 ml of water and then 52.5 ml of 6N hydrochloric acid. The suspension was heated at reflux for 10 minutes to give a slurry which was chilled in an ice bath for 30 minutes. The solid was collected washed with 750 ml of water and dried to give crude 6-chlorocarbazole-2-glyoxalic acid as 67.5 g of red-orange solid.

A suspension of 27.2 g of magnesium in 1125 ml of tetrahydrofuran was degassed and placed under nitrogen. 80 ml of methyl bromide was distilled into the flask over 1.0 hour. After about 10 minutes the flask was chilled in an ice bath and the methyl bromide flow was controlled to maintain an internal temperature of 20 to 28°. The mixture was then stirred another 1.0 hour without cooling.

A turbid solution of crude 6-chlorocarbazole-2-glyoxalic acid in 750 ml of tetrahydrofuran was degassed, placed under nitrogen and cooled in an ice bath as the methylmagnesium bromide solution was added over 1.5—2.0 hours at a rate that maintained an internal temperature of 8° (gas evolution). The ice bath was left in place as the reaction mixture slowly came to room temperature overnight. The mixture was recooled in an ice bath and treated over 30 minutes with 250 ml of 6N hydrochloric acid at a rate such that the temperature was 15°. The mixture was treated with 500 ml of saturated brine, stirred without cooling for 30 minutes, filtered and washed with 200 ml of tetrahydrofuran. The aqueous layer of the filtrate was extracted with 200 ml of tetrahydrofuran. The combined organic solutions were evaporated at 60° until free of tetrahydrofuran. The residue was suspended in 1000 ml of water. This suspension was stirred for 1.0 hour to give a finely divided suspension which was filtered. The solid was washed with 4000 ml of water and dried to give 72.8 g of product containing 94.1% of 6-chloro-α-hydroxy-α-methylcarbazole-2-acetic acid 5.4% of 6-chloro-α-methylene-2-carbazole acetic acid and 0.5% of a compound of unknown structure.

To a suspension of the crude 6-chlor-α-hydroxy-α-methylcarbazole-2-acetic acid and 6-chloro-α-methylene-2-carbazole acetic acid in 1685 ml of glacial acetic acid was added 225 g of stannous chloride dihydrate. The mixture was degassed, placed under nitrogen, treated with 470 ml of concentrated hydrochloric acid and heated at 40° for 22 hours. After 4—5 hours virtually all the 6-chloro-α-hydroxy-α-methylcarbazole-2-acetic acid had been converted to crude 6-chloro-α-methylcarbazole-2-acetic acid. To the solution of the 6-chloro-α-methylene-2-carbazole acetic acid and crude 6-chloro-α-methylcarbazole-2-acetic acid was added 185 mg of platinum oxide. The resulting solution was loaded with the aid of 50 ml of acetic acid into a glass liner, pressurized to 500 psi with hydrogen and heated at 40° with rocking for 22 hours. The solution was removed from the liner with the aid of 50 ml of acetic acid and diluted with 3750 ml of water. The resultant suspension was stirred under cooling for 2.0 hours and filtered. The solid was washed with 2000 ml of 2N hydrochloric acid followed by 6000 ml of water and dried to give 65 g of crude 6-chloro-α-methylcarbazole-2-acetic acid, m.p. 207.5—208°C.

EXAMPLE 4

A stirred solution of 5 g of carbazole, 50 ml of chloroform, 5 ml of acetic anhydride and 3 drops of concentrated sulfuric acid was heated under reflux (dry nitrogen atmosphere) for 5 hours. The reaction mixture was concentrated to dryness under reduced pressure and the residue was partitioned between ether and water. After the water was separated, the organic phase was washed by extraction with water, dilute sodium bicarbonate and water again. Following drying of the ether solution over anhydrous magnesium sulfate, the desiccant was removed by filtration and the ether was evaporated; yielding 6.1 g of 9-acetylcarbazole, m.p. 76—77°.

Over the course of 2 hours, a solution of 20 g of 9-acetylcarbazole, 16 g of ethyloxalylchloride and 120 ml of methylene chloride was added dropwise to a stirred, cooled (+3°) mixture of 60 g of anhydrous aluminum chloride and 200 ml of methylene chloride under an atmosphere of dry nitrogen. After the addition the reaction mixture was stirred for 1 hour with the temperature going from +3 to +10°, heated at reflux for 1/2 hour and poured onto a mixture of ice and concentrated hydrochloric acid. The aqueous mixture was extracted with 1200 ml of methylene chloride. The combined extracts were washed by extraction with 1600 ml of water and dried over anhydrous sodium sulfate. Following filtration of the desiccant and evaporation of methylene chloride, a residue of 28.3 g was obtained. The residue was triturated with ether, filtered and dried to give 21.2 g (71.4%) of 9-acetylcarbazole-2-glyoxalic acid ethyl ester, m.p. 101—107°.

EXAMPLE 5

A stirred solution of 6 g of 9-acetylcarbazole-2-glyoxalic acid ethyl ester, 150 ml of methanol and 2 ml of concentrated hydrochloric acid (12N) was heated at reflux for 5 hours and concentrated under reduced pressure to dryness. The residue was crystallized from methanol to give 4.6 g (93.6%) of carbazole-2-glyoxalic acid methyl ester, m.p. 191—193°.

EXAMPLE 6

A solution of 1.1 ml of sulfuryl choride and 60 ml 1,2-dichloroethane was added dropwise to a stirred, refluxing solution of 2.8 g of carbazole-2-glyoxalic acid methyl ester in 300 ml of 1,2-dichloroethane (under an atmosphere of dry nitrogen). After the addition the reaction mixture was refluxed and stirred 20 minutes. The reaction mixture was concentrated to dryness under reduced pressure. The residue was partitioned between ether and water. After the ethyl layer was separated, it was washed by extraction with water, dilute sodium bicarbonate and again water. Following drying of the ether solution over anhydrous magnesium sulfate, the desiccant was removed by filtration and the ether evaporated, yielding 3.0 g. Recrystallization from chloroform gave 1.75 g (54.8%). A second recrystallization from methanol yielded 1.3 g of 6-chlorocarbazole-2-glyoxalic acid methyl ester, m.p. 191—193°.

EXAMPLE 7

Over the course of 1 hour, a solution of 2.9 g of methylmagnesium iodide, made from 0.42 g of magnesium and 4.2 g of methyl iodide (excess, in 40 ml of ether was added dropwise to a cooled (+2°) stirred solution of 2 g of 6-chlorocarbazole-2-glyoxalic acid methyl ester in 100 ml of tetrahydrofuran. After the addition, the reaction mixture was stirred with no cooling for 1 hour, heated at reflux for 30 minutes and again cooled in an ice water bath. Water (60 ml) containing 4 ml of concentrated hydrochloric acid was added dropwise. The mixture was saturated with sodium chloride and extracted with ether (5 x 100 ml). The combined ether extracts were washed by extraction with water and dried over anhydrous magnesium sulphate. Following filtration of desiccant and evaporation of the ether, a residue of 2.5 g remained. The residue was dissolved in 100 ml of ethanol and 20 ml of 3N sodium hydroxide was added. After heating at reflux for 1 hour, the reaction solution was concentrated to dryness under reduced pressure. The residue was dissolved in 900 ml of water and filtered. A slight excess of concentrated hydrochloric acid was added to the filtrate and the liberated acid was extracted with ether (3 x 200 ml). The combined ether extract was washed by extraction with water and dried over anhydrous magnesium sulfate. Following filtration of the desiccant and evaporation of the ether, 1.5 g of solid remained. The solid was triturated with chloroform and filtered, yielding 1.2 g (59.6%). Recrystallization of 400 mg of the crude acid afforded 206 mg of 6-chloro-α-hydroxy-α-methylcarbazole-2-acetic acid, m.p. 218—220°.

EXAMPLE 8

A stirred solution of 0.4 g of 6-chloro-α-hydroxy-α-methylcarbazole-2-acetic acid, 20 ml of tetrahydrofuran and 0.8 ml of concentrated sulfuric acid was heated at reflux for 22 hours. The reaction mixture was poured onto a mixture of ice and water and extracted with 300 ml of ether. The ether extract was washed by extraction with water and dried over anhydrous magnesium sulfate. Filtration of the desiccant, evaporation of the ether and crystallization from ethyl acetate gave 0.1 g (12.0%) product. Recrystallization from methanol afforded 45 mg of 6-chloro-α-methylenecarbazole-2-acetic acid, m.p. 233—234° (dec.).

EXAMPLE 9

A mixture of 40 mg of 6-chloro-α-methylenecarbazole-2-acetic acid, 50 ml of ethanol and 30 mg of platinum oxide was shaken in an atmosphere of hydrogen at room temperature. After 7 hours, the catalyst was filtered from the mixture and following evaporation of the ethanol, the residue was crystallized from chloroform, yielding 13 mg (32.3%) of 6-chloro-α-methylcarbazole-2-acetic acid, m.p. 184—186° (dec.).

**Claims**

1. A process for the preparation of 6-chloro-α-methylcarbazole-2-acetic acid which comprises a) either treating the compound of the formula

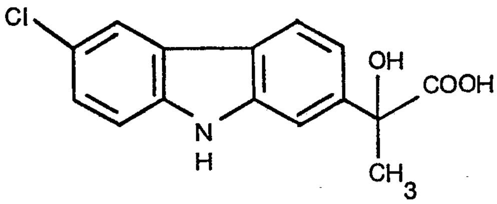

IIIb

with a dehydrating agent in an inert organic solvent and

b) hydrogenating the obtained compound of formula

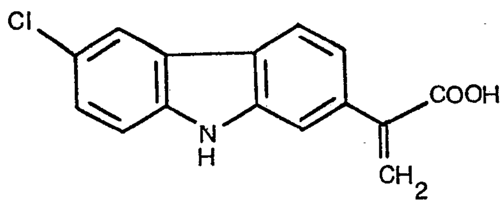

IV.

or

c) subjecting the compound of formula IIIb to hydrogenolysis and
d) recovering the product of step b) or c) of the formula

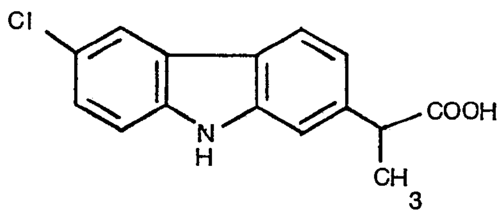

V

2. A process for the preparation of the compound of the formula

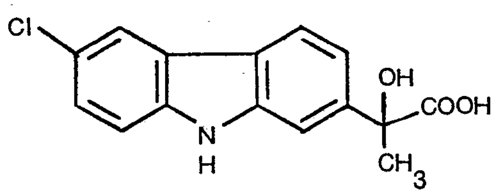

IIIb

which process comprises

a) chlorinating a compound of the formula

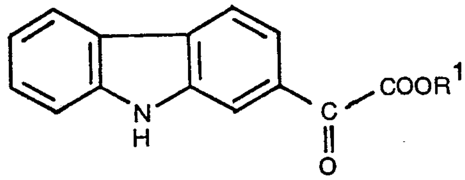

IIb

wherein $R^1$ is methyl or ethyl, in an inert organic solvent at a temperature in the range of from about −40 to −60°C and ether

b) treating the obtained compound of formula

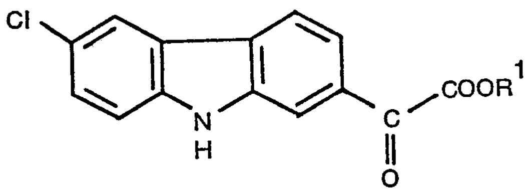

IIc

wherein $R^1$ is as above,

with a methyl Grignard reagent or with methyllithium in an inert organic solvent and

c) treating the obtained compound of formula

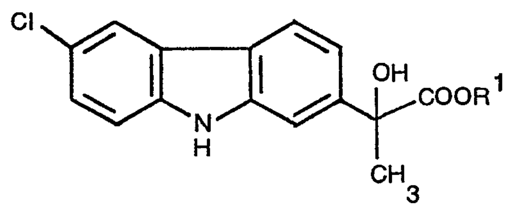

IIIa

wherein $R^1$ is as above,

with a base in an inert organic solvent, or

d) treating the ester of formula IIc with a base and

e) treating the obtained acid of formula

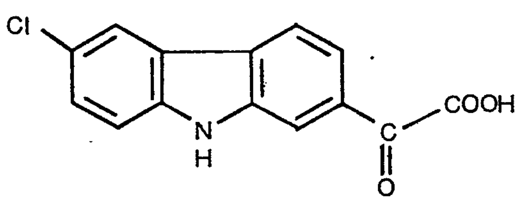

IId

with a methyl Grignard or with methyllithium.

3. A process according to claim 2, in which a compound of formula IIb is treated with a chlorinating agent at a temperature in the range of from about −40 to −60°C.

4. Compounds of the formula

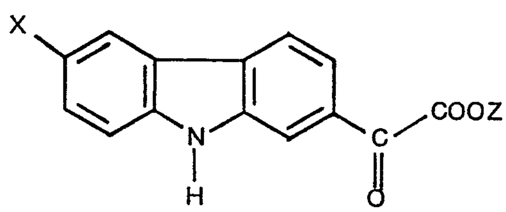

II

wherein X is hydrogen or chlorine and Z is hydrogen, methyl or ethyl.

5. A compound according to claim 4, 6-chlorocarbazole-2-glyoxalic acid ethyl ester, 6-chlorocarbazole-2-glyoxalic acid and 6-chlorocarbazole-2-glyoxalic acid methyl ester.

6. Compounds of the formula

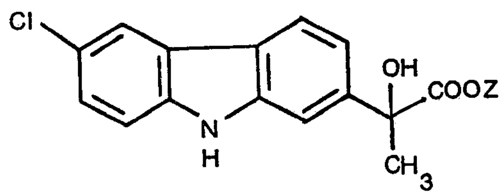

III

wherein Z is hydrogen, methyl or ethyl.

7. 6-Chloro-α-hydroxy-α-methylcarbazole-2-acetic acid.

8. 6-Chloro-α-methylenecarbazole-2-acetic acid.

**Revendications**

1. Un procédé de préparation de l'acide 6-chloro-α-méthylcarbazole-2-acétique qui consiste:

a) soit à traiter le composé de formule:

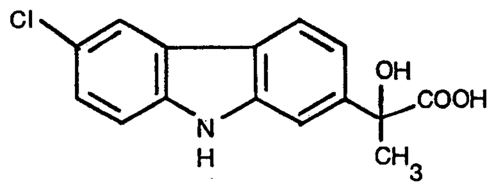

IIIb

par un agent déshydratant dans un solvant organique inerte; et

b) à hydrogéner le composé obtenu de formule:

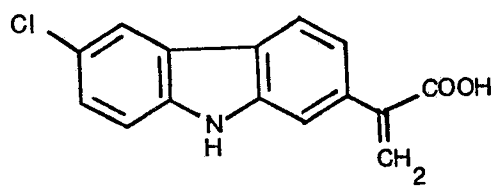

IV

soit

c) à soumettre le composé de formule IIIb à une hydrogénolyse et

d) à recueillir le produit obtenu au stade b) ou c), de formule

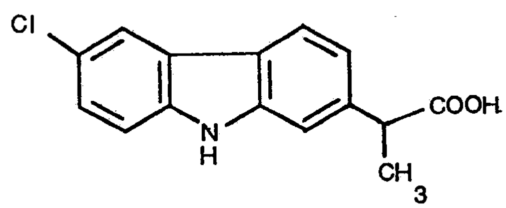

V

2. Un procédé de préparation du composé de formule:

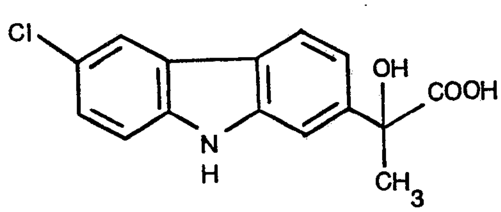

IIIb

procédé qui consiste:

a) à chlorer un composé de formule:

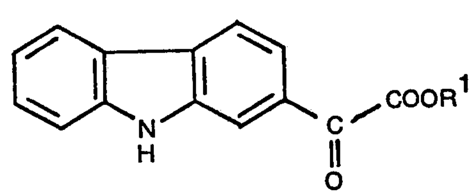

IIb

dans laquelle $R^1$ est un groupe méthyle ou éthyle, dans un solvant organique inerte à une température dans l'intervalle d'environ —40 à —60°C et soit

b) à traiter le composé obtenu de formule:

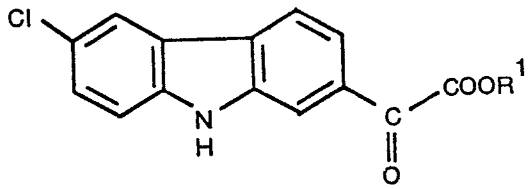

IIc

dans laquelle $R^1$ a les significations indiquées cidessus, par un réactif méthylé de Grignard ou par le méthyl-lithium dans un solvant organique inerte; et

c) à traiter le composé obtenu de formule:

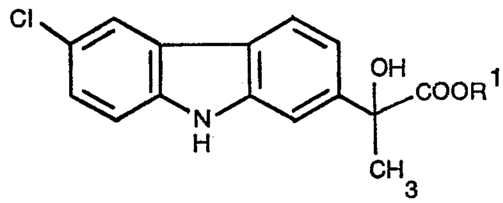

IIIa

dans laquelle $R^1$ a les significations indiquées cidessus, par une base dans un solvant organique inerte, soit

d) à traiter l'ester de formule IIc par une base et

e) à traiter l'acide obtenu de formule:

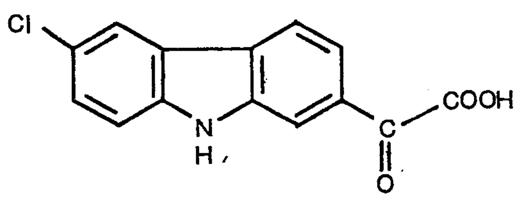

IId

par un réactif méthylé de Grignard ou par le méthyllithium.

3. Un procédé selon la revendication 2, dans lequel on traite un composé de formule IIb par un agent chlorant à une température dans l'intervalle allant d'environ —40 à —60°C.

4. Composés de formule:

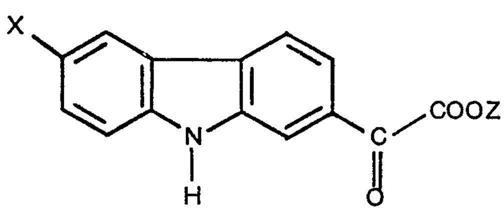

II

dans laquelle X représente l'hydrogène ou le chlore et Z représente l'hydrogène, un groupe méthyle ou éthyle.

5. Un composé selon la revendication 4, l'ester éthylique de l'acide 6-chlorocarbazole-2-glyoxalique, l'acide 6-chlorocarbazole-2-glyoxalique et l'ester méthylique de l'acide 6-chlorocarbazole-2-glyoxalique.

6. Composés de formule:

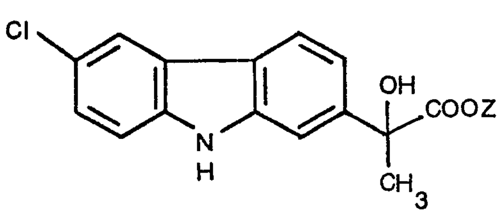

III

dans laquelle Z représente l'hydrogène, un groupe méthyle ou éthyle.

7. L'acide 6-chloro-$\alpha$-hydroxy-$\alpha$-méthylcarbazole-2-acétique.

8. L'acide 6-chloro-$\alpha$-méthylène-carbazole-2-acétique.

**Patentansprüche**

1. Ein Verfahren zur Herstellung der 6-Chlor-$\alpha$-methylcarbazol-2-essigsäure, dadurch gekennzeichnet, dass man

a) entweder eine Verbindung der Formel

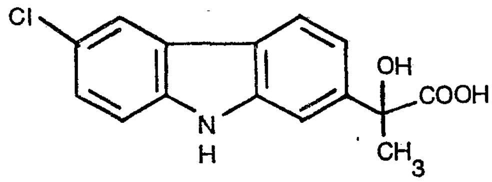

IIIb

mit einem Dehydratisierrungsmittel in einem inerten organischen Lösungsmittel behandelt und

b) die erhaltene Verbindung der Formel

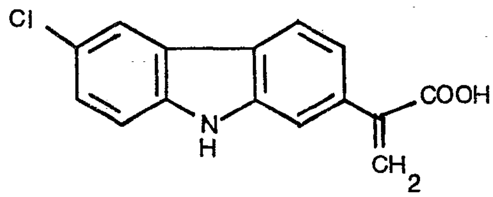

IV

hydriert oder

c) die Verbindung der Formel III b der Hydrogenolyse unterwirft und
d) das Produkt der Stufe b) oder c) der Formel

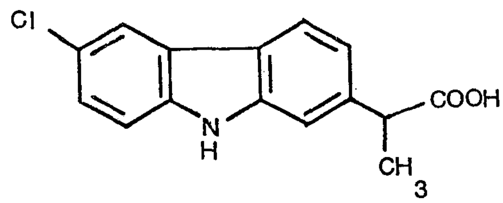

V

gewinnt.

2. Verfahren zur Herstellung einer Verbindung der Formel

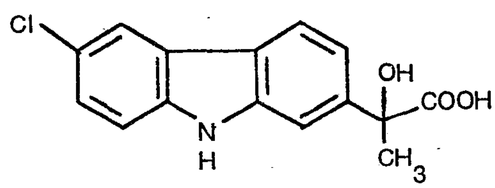

IIIb

dadurch gekennzeichnet, dass man

a) eine Verbindung der Formel

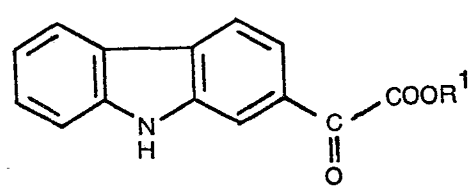

IIb

worin $R^1$ Methyl oder Aethyl ist, in einem inerten organischen Lösungsmittel bei einer Temperatur im Bereich von etwa —40 bis —60°C chloriert und entweder.

b) die erhaltene Verbindung der Formel

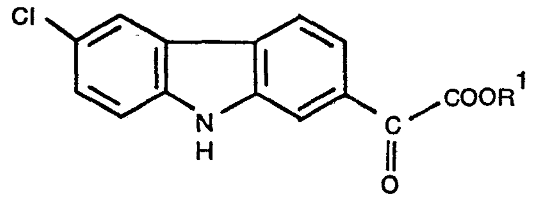

IIc

worin $R^1$ wie oben ist, mit einem Methyl-Grignard-Reagenz oder mit Methyllithium in einem inerten organischen Lösungsmittel behandelt und

c) die erhaltene Verbindung der Formel

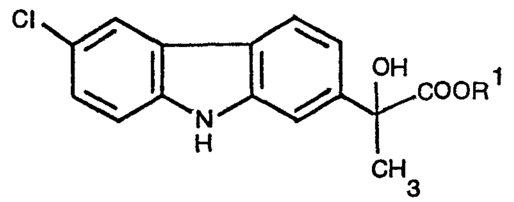

IIIa

worin $R^1$ wie oben ist, mit einer Base in einem inerten organischen Lösungsmittel behandelt oder

d) den Ester der Formel II c mit einer Base Behandlet und
e) die erhaltene Säure de Formel

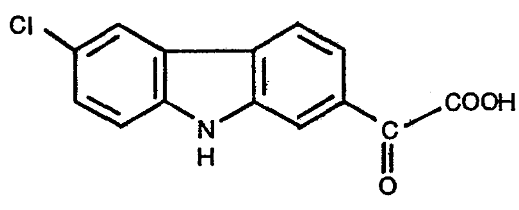

IId

mit einem Methyl-Grignard oder mit Methyllithium behandelt.

3. Ein Verfahren nach Anspruch 2, in dem eine Verbindung der Formel II b mit einem Chlorierungsmittel bei einer Temperatur im Bereich von etwa —40 bis —60°C behandelt wird.

4. Verbindungen der Formel

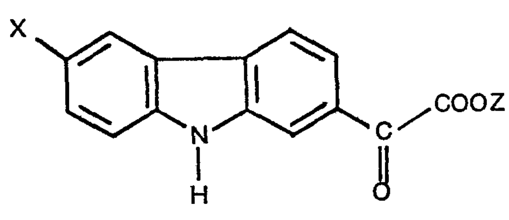

II

worin X Wasserstoff oder Chlor und Z Wasserstoff, Methyl oder Aethyl ist.

5. Eine Verbindung nach Anspruch 4, 6-Chlorocarbazol-2-glyoxalsäureäthylester, 6-Chlorcarbazol-2-glyoxalsäure und 6-Chlorcarbazol-2-glyoxalsäuremethylester.

6. Verbindungen der Formel

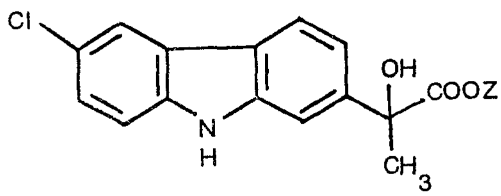

III

worin Z Wasserstoff, Methyl oder Aethyl ist.

7. 6-Chlor-$\alpha$-hydroxy-$\alpha$-methylcarbazol-2-essigsäure.
8. 6-Chlor-$\alpha$-methylenecarbazol-2-essigsäure.